(19) Europäisches Patentamt
European Patent Office
Office européen des brevets

(11) Publication number: **0 151 477**
**B1**

(12) # EUROPEAN PATENT SPECIFICATION

(45) Date of publication of patent specification: **12.09.90**

(51) Int. Cl.$^5$: **C 07 D 409/12,**
A 61 K 31/415, A 01 N 43/50

(21) Application number: **85101086.8**

(22) Date of filing: **01.02.85**

(54) **1H-Imidazole derivatives, a process for preparing them and pharmaceutical compositions containing them.**

(30) Priority: **02.02.84 ES 529608**
**01.06.84 ES 533353**
**06.08.84 ES 535656**

(43) Date of publication of application:
**14.08.85 Bulletin 85/33**

(45) Publication of the grant of the patent:
**12.09.90 Bulletin 90/37**

(84) Designated Contracting States:
**BE CH DE FR GB IT LI NL**

(56) References cited:
**EP-A-0 004 917**
**EP-A-0 019 133**
**EP-A-0 038 972**
**EP-A-0 073 663**
**EP-A-0 123 218**
**DE-A-2 917 244**
**DE-B-1 940 388**
**US-A-2 692 884**

**The file contains technical information submitted after the application was filed and not included in this specification**

(73) Proprietor: **FERRER INTERNACIONAL, S.A.**
**Gran Via Carlos III, 94**
**08028 Barcelona (ES)**

(72) Inventor: **Foguet, Rafael**
**Llusanés 8**
**ES-08028 Barcelona (ES)**
Inventor: **Moreno, Marcial, Dr.-Chem.**
**Sabadell 23**
**S. Cugat Barcelona (ES)**
Inventor: **Raga, Manuel**
**Sors 17-20**
**Barcelona 08024 (ES)**
Inventor: **Cuberes, M. Rosa**
**Barcelona 2**
**S. Cugat Barcelona (ES)**
Inventor: **Castelló, Josep M.**
**Principe de Asturias 35**
**ES-08012 Barcelona (ES)**
Inventor: **Ortiz, José A., Dr.-Med.**
**Córcega 429**
**ES-08037 Barcelona (ES)**

(74) Representative: **Kinzebach, Werner, Dr.**
**Patentanwälte Reitstötter, Kinzebach und Partner Sternwartstrasse 4 Postfach 86 06 49**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 151 477 B1**

Courier Press, Leamington Spa, England.

**Description**

The present invention relates to 1H-imidazole derivatives having antifungal activity.

DE—A—29 17 244 discloses compounds of th general formula:

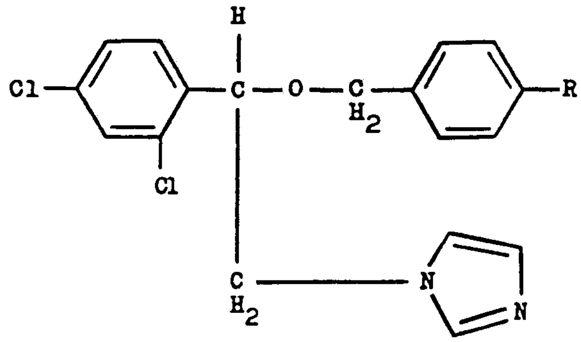

I

wherein R is phenyl or phenylthio. These compounds are described to be effective against fungi, yeasts and bacteria.

DE—A—19 40 388 discloses compounds of the general formula:

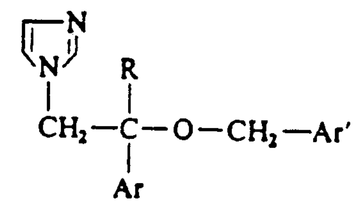

(I)

in which R is hydrogen or methyl, Ar is phenyl which may be substituted by one or two halogen atoms or a methyl group, and Ar' is phenyl which may be substituted by one or two halogen atoms or a methyl or methoxy group. These compounds are described to be fungicidal and bactericidal. Among these compounds is micronazole (R = H; Ar and Ar' = 2,4-dichlorophenyl) which is a well known and clinically effective drug in antifungal therapy.

EP—A—49 17 discloses compounds of the general formula:

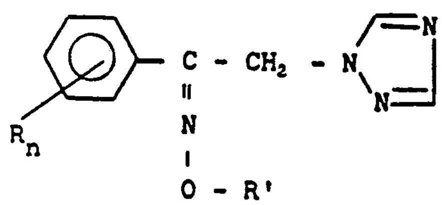

(I)

wherein R inter alia is halogen, R' is alkyl, alkenyl, alkynyl or optionally substituted benzyl or styryl and n is 0 to 3. These compounds are described to have fungicidal activity.

The present invention relates to 1H-imidazole derivates of the general formula (I):

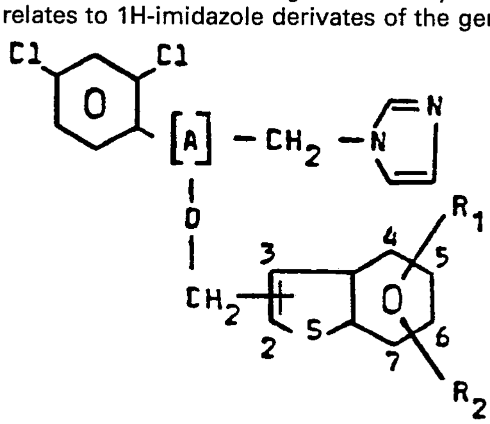

(I)

wherein [A] is an imino-

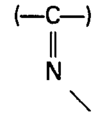

or methine-

$$\underset{|}{(-CH-)}$$

group,

$R_1$ and $R_2$, which may be the same or different, are hydrogen or halogen, the halogen being attached to the benzo[b]-thiophene group in the 2, 4, 5, 6 or 7 position and the methylene ($-CH_2-$) group being bonded to the benzo[b]-thiophene group in 2 or 3 position, and the nontoxic addition salts thereof, as well as to a process for preparing these compounds and to pharmaceutical compositions containing these compounds.

Preferably, the halogen is chloro, bromo or fluoro. The preferred addition salts are the mononitrates and heminaphthalene-1,5-disulphonates.

The compounds of the present invention may be prepared according to the following schema:

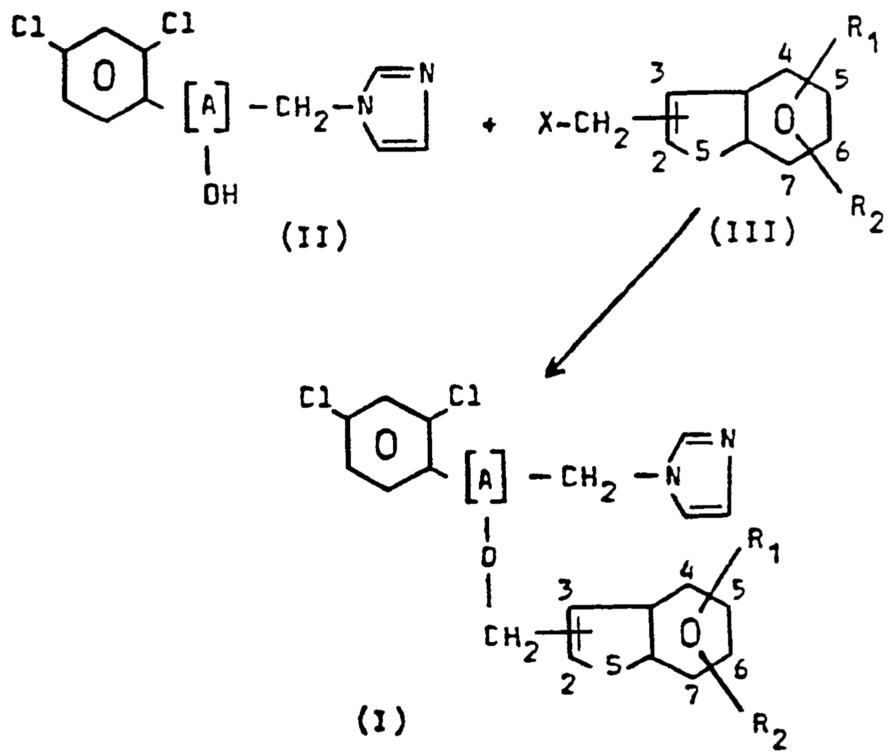

(II) (III) (I)

In the starting compounds of the general formula (II), [A] has the same meaning as stated above, and in the starting compounds of the general formula (III), X is chlorine or bromine, $R_1$ and $R_2$ have the same meaning as stated above and likewise the methylene group ($-CH_2-$) is bonded to the benzo[b]thiophene group in the same position as defined above.

The reaction between the oxime or the alcohol of the general formula (II) and the halides of the general formula (III) occurs conveniently in the presence of a suitable mineral base strong enough to ionize the compounds of the general formula (II), such as hydroxides, or alkaline or alkaline-earth hydrides. Potassium hydroxide and sodium hydride are preferably employed.

The reaction is advantageously carried out at a wide temperature range (from −55°C to 100°C) and in an aprotic solvent selected from an alkanone with up to 6 carbon atoms or a polyalkylated amide. Acetone or hexamethylphosphortriamide are preferably employed.

The addition salts are prepared according to standard procedures by treating the compounds (I) as free base with the desired acid, such as inorganic acids, for esample hydrochloric, sulfuric, nitric or phosphoric acid, and organic acids, for example tartaric, citronic, lactic or heminaphthalen-1,5-disulfonic acid. The reaction may be carried out in an alkanol or alkanone having 1 to 4 carbon atoms or mixtures thereof with water. Suitable solvents are for example methanol, i-propanol, n-propanol, methylethylketone. Preferred are ethanol, n-butanol or acetone.

The reaction steps as described so far lead to the compounds of the general formula (I) according to the present invention.

The oxime and alcohol used as starting material of the general formula (II) are already known and are obtained according to the processes described by Von. G. Mixich and K. Thiele (Arzneimittel-Forschung, 29(II), Nr. 10, 1510—3, 1979) — Oxime, Z configuration — and by E. F. Godefroi et al (J. Med. Chem., 12, 784—9, 1969) — Alcohol — for example by treating 1-(2,4-dichlorophenyl)-2-(1H-imidazol-1-yl)ethanone with hydroxylamine or sodium borohydride respectively.

The halides of the general formula (III) are obtained by known processes of organic chemistry, for example:

a) from Benzo[b]thiophene either by chloromethylation (R. Neidlen and E. P. Mrugowski: Arch. Pharm. (Weinheim, Ger.), 308(7), 513—9, 1975):

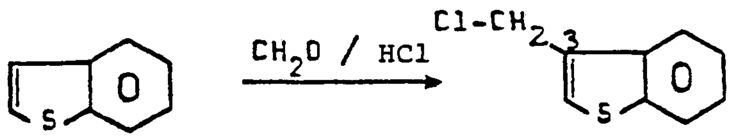


(III, X=Cl, $R_1$=$R_2$=H, 3 position)

or by methylation and final bromination (D. A. Shirley and M. D. Cameron: JACS, 74, 664, 1951; US Patent No. 4,282,227):

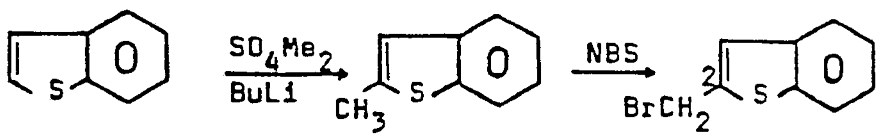


(III, X=Br, $R_1$=$R_2$=H, 2 position)

b) from 2-chloro-3-methylbenzo[b]thiophene by bromination (European Patent No. 54,233):

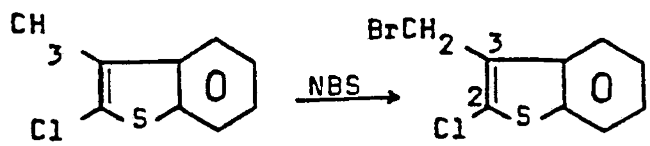


(III, X=Br, $R_1$=2-Cl, $R_2$=H, 3 position)

and 2-chloro-3-methylbenzo[b]thiophene is in turn obtained by chlorination of 3-methylbenzo[b]thiophene (V. I. Dronov et al: CA, 79, 115388f):

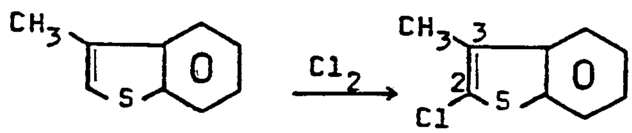


(III, X=H, $R_1$=2-Cl, $R_2$=H, 3 position)

c) from the corresponding thiophenol by total synthesis, for example, 2-chloro-2-propenyl-phenylthioether is formed by treating 2,3-dichloropropene, transposition in N,N-diethylaniline and cyclization with concentrated hyrochloric acid (W. K. Anderson and E. J. La Voie: J. Chem. Soc., Chem. Commun., (5), 174, 1974; W. K. Anderson et al: J. Chem. Soc., Perkin Trans. 1, (1), 1—4, 1976):

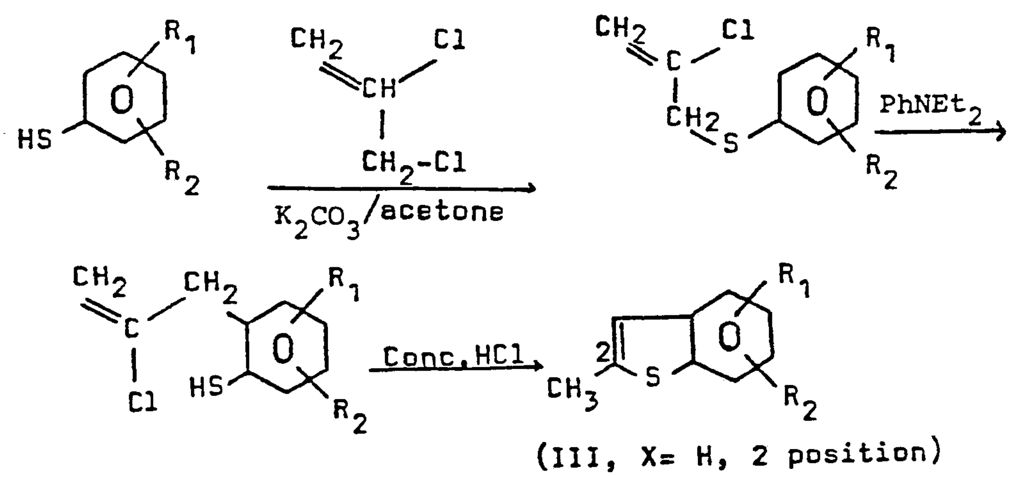


(III, X= H, 2 position)

or by an alternative process — also from the corresponding thiohenol by total synthesis — for example by forming the respecive 2-phenylthiopropionaldehyde acetal by treatment with 2-bromopropionaldehyde in sodium ethoxide and cyclization with a mixture of polyphosphoric acid and phosphorous pentoxide (Yaksuo Matsuki and Fusaja Shoji: Nippon Kagaku Zasshi, 86(10), 1067—72., 1965):

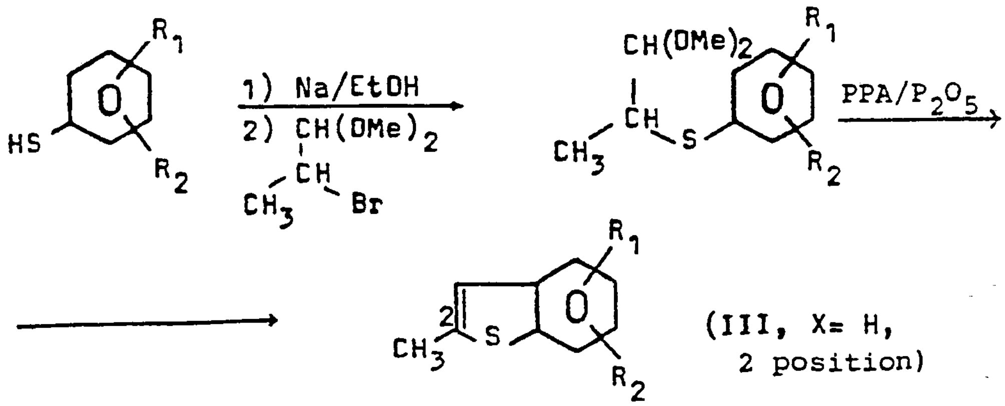


(III, X= H, 2 position)

d) in a similar manner, the 3-position compounds are also obtained from the corresponding thiophenol by total synthesis and forming the respective phenylthioacetone by treatment with chloroacetone and cyclization with polyphosphoric acid (N.B. Chapman et al: J. Chem. Soc. C., (5), 512—22, 1968; N. B. Chapman et al: Ibid, (5), 2747—51, 1968):

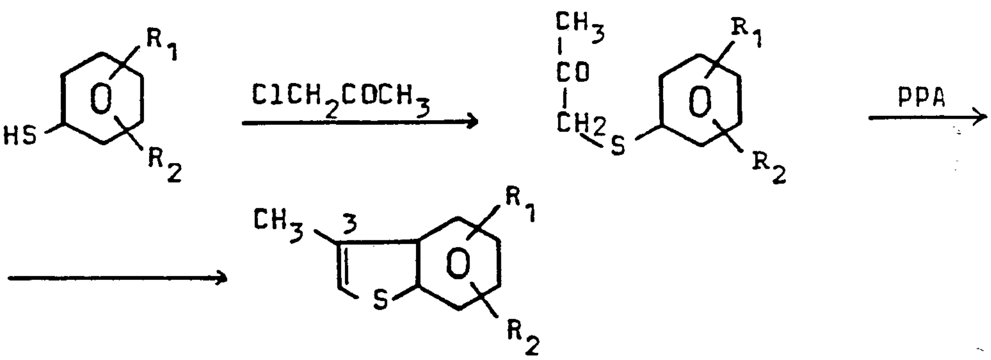


(III, X=H, 3 position)

PPA: Polyphosphoric acid
NBS: N-Bromosuccinimide

Halogenation of intermediates (III, X=H) with N-bromosuccinimide or with N-chlorosuccinimide in carbon tetrachloride, under the conditions described by N. B. Chapman et al (J. Chem. Soc. C., (5), 512—22, 1968) and N. B. Chapman et al (Ibid, (5), 2747—51, 1968), leads to the intermediates (III, X=Br or Cl).

The starting thiophenol, when $R_1$ or $R_2$ are halogen in metaposition, leads to corresponding benzo[b]thiophenes substituted in 4- and 6-position. The isomers can be separated by customary methods of organic chemistry; column chromatrography is preferred.

The compounds of the present invention have antifungal activity, thus becoming a powerful medication against infections caused by fungi and yeast, both in man and (pet)animals. They are also suitable for combating crop diseases caused by the above infectious microorganisms.

Table 1 shows minimal inhibitory concentrations (MIC) (expressed un µg/ml) found for two preferred compounds versus Miconazole mononitrate. The respective readings were effected at 4 days (1—14 yeast microorganism) or at 5 days (15—20 fungus microorganism).

TABLE 1

| Microorganism | MIC (µg/ml) Compound Ex.3 | Compound Ex.6 | Miconazole mononitrate |
|---|---|---|---|
| 1. Candida albicans HM1 | 16 | 8 | 8 |
| 2. " " HM2 | 4 | 4 | 4 |
| 3. " " HM3 | 4 | 4 | 8 |
| 4. " " HM4 | 4 | 4 | 8 |
| 5. Candida guillermondii | 2 | 2 | 1 |
| 6. Candida albicans s.p. | 4 | 4 | 4 |
| 7. Criptococus n. | 0,25 | 0,5 | 0,25 |
| 8. Sacharomyces C.ATCC E-9763 | 8 | 8 | 8 |
| 9. " " 36375 | 0,5 | 0,5 | 0,25 |
| 10. Candida Trop. ATCC 13803 | 16 | 8 | 8 |
| 11. Rodoturule rubra ATCC E-9449 | 4 | 8 | 8 |
| 12. Turulopsis glab. ATCC 15-126 | 4 | 8 | 4 |
| 13. Candida alb. ATCC E-10231 | 4 | 8 | 8 |
| 14. Candida pseudotr. ATCC 14245 | 0,25 | 0,25 | 0,25 |
| 15. Aspergillus fumigatus HM5 | 2 | 4 | 4 |
| 16. " " HM6 | 2 | 2 | 8 |
| 17. " niger HNE | 1 | 2 | 4 |
| 18. " " HNE | 2 | 32 | 16 |
| 19. " " s.p. | - | 4 | 4 |
| 20. " " 16.404 | 2 | 16 | 16 |

The compound of the present invention mixed with pharmaceutically acceptable carriers can be administered by the oral route to humans and animals in the form of tablets, capsules, coated tablets, syrups, solutions, powder, etc., by injectable route, by rectal route, and by vaginal-intrauterine route in the form of a ovulum, vaginal tablet, ointment, cream, pessary, lotion, etc., at daily doses ranging from 100 to 800 mg; and by topical route in the form of a cream, lotion, ointment, emulsion, solution, shampoo, powder, gel, etc. at concentrations ranging from 0,1 to 5%.

Also the compounds of the present invention in admixture with a diluent or carrier, when used against crop diseases, can be administered by watering, atomizing, spraying, dusting, or in the form of a powder, cream, paste, etc. at the rate of 0,1—15 kg per hectare of soil.

Some examples are described hereinbelow referring to the possible way to obtain (I) by following the steps of the disclosed process.

### Example 1

1-(2,4-Dichlorophenyl)-2-(1H-imidazol-1-yl)ethanone (Z)-O-[(3-benzo[b]thienyl)methyl]oxime

(I, [A] = —C—, $R_1$ = $R_2$ = H, 3 position)
‖
N
\

In a 25-ml flash 1.89 g (0.0070 mol) of 1-(2,4-dichlorophenyl)-2-(1H-imidazol-1-yl)ethanone(Z)oxime are suspended in 6 ml of acetone, then under vigorous stirring 0.507 g (0.0077 mol) of 85% triturated potassium hydroxide are added. The mixture is stirred for 1 hour at room temperature and then 1.41 g (0.0077 mol) of 3-chlormethylbenzo[b]thiophene are added and left to stand overnight at room temperature under stirring. The solvent is evaporated in vacuo, the residue is dissolved in chloroform and washed neutral with water, then dried over sodium sulphate and evaporated. The crude material thus obtained is purified by silicagel column chromatography, yielding 1.58 g (54%) of 1-(2,4-dichlorophenyl)-2-(1H-imidazol-1-yl)ethanone(Z)-O-(3-benzo[b]thienyl)methyl oxime (oily product).

$^1$H-NMR ($CDCl_3$): δ 5.20 (s, 2H), 5.80 (s, 2H), 6.70 (s, wide, 1H), 6.95 (s, wide, 1H), 7.0—8.15 (m, 9H).

### Example 2

Bis[1-(2,4-dichlorophenyl)-2-(1H-imidazol-1-yl)ethanone] (Z)-O-[(3-benzo[b]thienyl)methyl]oxime

(I, [A] = —C—, $R_1$ = $R_2$ = H, 3 position, ½ $C_{10}H_8O_6S_2$)
‖
N
\

A solution of 0.100 g (0.00024 mol) of the compound as obtained in Example 1 in 3 ml of ethanol is added to a solution of 0.050 g (0.000132 mol) of 1,5-naphthalenedisulphonic acid (75.8%) in 5 ml of ethanol. 0.060 g of Bis[1-(2,4-dichlorophenyl)-2-(1H-imidazol-1-yl)ethanone (Z)-O-[(3-benzo[b]thienyl)methyl]oxime naphthalen-1,5-disulphonate. Melting point 206—208°C. Yield 45%.

### Example 3

1-[2-[(2-Benzo[b]thienyl)methoxy]-2-(2,4-dichlorophenyl)ethyl]-1H-imidazol

(I, [A] = —CH—, $R_1$ = $R_2$ = H, 2 position)
|

A 100 ml flask previously subjected to an inert and dry atmosphere is charged with a suspension of 0.0925 g (0.00212 mol) of sodium hydride (55%) in paraffin oil, which is then washed three times with 5 ml of anhydrous hexamethylphosphorotriamide. Thereafter, 5 ml of hexamethylphosphorotriamide are added, the mixture is cooled to 0°C and then a solution of 0.521 g (0.00202 mol) of α-(2,4-dichlorophenyl)-1H-imidazol-1-ethanol in 7 ml of hexamethylphosphorotriamide is added. The mixture is stirred at room temperature until the evolution of hydrogen stops, and then allowed to stand at 50°C for 1 hour. Then, the mixture is cooled again to 0°C and a solution of 0.470 g (0.00207 mol) of 2-bromomethylbenzo[b]thiophene in 5 ml of hexamethylenephosphorotriamide is added in the course of 15 minutes. The mixture is allowed to stand at room temperature for 5 hours under stirring. After adding water and diethyl ether, the aqueous phase is extracted several times with ether, the organic phases are washed neutral with water, dried over sodium sulphate and evaporated in vacuo. The residue is purified by silicagel column chromatography. 0.323 g of 1-[2-[(2-benzo[b]thienyl)methoxy]-2-(2,4-dichlorophenyl)ethyl]-1H-imidazol (oily product) are obtained. Yield 40%.

$^1$H-NMR ($CDCl_3$): δ 4.04—4.25 (m, 2H), 4.35—4.85 (two d, 2H), 4.95—5.20 (m, 1H), 6.9 (s wide, 1H), 7.08—8.0 (m, 1 OH).

### Example 4

1-[2-[(3-Benzo[b]thienyl)methoxy]-2-(2,4-dichlorophenyl)ethyl]-1H-imidazol

(I, [A] = —CH—, $R_1$ = $R_2$ = H, 3 position)
|

A 100 ml flask previously subjected to an inert and dry atmosphere is charged with a suspension of 0.142 g (0.00325 mol) of sodium hydride in paraffin oil (55%) which is then washed three times with 5 ml of anhydrous hexamethylphosphorotriamide. Thereafter, 5 ml of hexamethylphosphorotriamide are added, the mixture is cooled to 0°C and then a solution of 0.771 (0.0030 mol) of α-(2,4-dichlorophenyl)-1H-imidazol-1-ethanol in 10 ml of hexamethylphosphorotriamide is added. The mixture is stirred at room temperature until the evolution of hydrogen stops, and then allowed to stand at 50°C for 1 hour. Then, the mixture is cooled again to 0°C and a solution of 0.595 g (0.00326 mol) of 3-chloromethylbenzo[b]thiophene in 10 ml of hexamethylphosphorotriamide is slowly added, and allowed to stand at room temperature for 5 hours

under stirring. After adding water and diethyl ether, 0.805 g of 1-[2-[(3-benzo[b]thienyl)methoxy]-2-(2,4-dichlorophenyl)ethyl]-1H-imidazol are obtained. Melting point 165—167°C. Yield 67%.

$^1$H-NMR ($CDCl_3$): δ 3.7—4.3 (m, 2H), 4.4—4.8 (two d, 2H), 4.9—5.1 (m, 1H), 6.7 (s, 1H), 6.85 (s, 1H), 7.0—7.8 (m, 9H).

Example 5

1-[2-[(3-Benzo[b]thienyl)methoxy]-2-(2,4-dichlorophenyl)ethyl]-1H-imidazol mononitrate

(I, [A] = —CH—, $R_1$ = $R_2$ = H, 3 position $NO_3H$)
|

A solution of 0.804 g (0.00199 mol) of the compound as obtained in Example 4 in 10 ml of b-butanol is added to 0.73 ml of 3N nitrid acid. 0.437 g of 1-[2-[(3-benzo[b]thienyl)methoxy]-2-(2,4-dichlorophenyl)ethyl]-1H-imidazol mononitrate crystallize at 0°C. Melting point 127—129°C. Yield 47%.

$^1$H-NMR ($CDCl_3$): δ 4.25—5.20 (m, 5H), 6.85 (s, 1H), 7.2—8.0 (m, 9H), 9.1 (s, 1H).

Example 6

1-[2-[(4-Chloro-3-benzo[b]thienyl)methoxy]-2-(2,4-dichlorophenyl)ethyl]-1H-imidazol

(I, [A] = —CH—, $R_1$ = 4—Cl, $R_2$ = H, 3 position)
|

A 100 ml flask previously subjected to an inert and dry atmosphere is charged with a suspension of 0.142 g (0.00325 mol) of sodium hydride in paraffin oil (55%) which is then washed three times with 5 ml of anhydrous hexamethylphosphorotriamide. Thereafter, 5 ml of hexamethylphosphorotriamide are added, cooled to 0°C and the mixture is then added to a solution of 0.771 g (0.0030 mol) of α-(2,4-dichlorophenyl)-1H-imidazol-1-ethanol in 10 ml of hexamethylphosphorotriamide. It is stirred at room temperature until the evolution of hydrogen stops, and allowed to stand at 50°C fo 1 hour. Thereafter, the mixture is cooled again to 0°C and a solution of 0.837 g (0.0032 mol) of 3-bromomethyl-4-chlorobenzo[b]thiophene in 10 ml of hexamethylenephosphorotriamide in the course of 15 minutes, and allowed to stand at room temperature for 5 hours under stirring. After adding water and diethyl ether to the mixture, the aqueous phase is extracted several times with ether, the organic phases are washed neutral with water, dried over sodium sulphate and evaporated in vacuo. The residue is purified by recrystallization from methylene chloride:diethyl ether giving 0.765 g of end product, still impurified with the starting alcohol, which is chromatographed over a silicagel column thus yielding 0.65 g (50%) of 1-[2-[(4-chloro-3-benzo[b]thienyl)methoxy]-2-(2,4-dichlorophenyl)ethyl]-1H-imidazol. Melting point 107—108°C.

$^1$H-NMR ($CDCl_3$): δ 3.9—4.4 (m, 2H), 418—5.1 (m, 2H), 5.0—5.2 (m, 1H), 6.9 (s, 1H), 7.0 (s, 1H), 7.1—7.5 (m, 7H), 7.65—7.85 (dd, 1H, J ca 7.5, 2.5Hz).

Example 7

1-(2,4-Dichlorophenyl)-2-(1H-imidazol-1-yl)ethanone] (Z)-O-[(2-chloro-3-benzo[b]thienyl)methyl]oxime

(I, [A] = —C—, $R_1$ = 2—Cl, $R_2$ = H, 3 position)
‖
N
\

In a 25-ml flash 1.35 g (0.0050 mol) of 1-(2,4-dichlorophenyl)-2-(1H-imidazol-1-yl)ethanone(Z)oxime are suspended in 10 ml of acetone, then 0.362 g (0.0055 mol) of 85% triturated potassium hydroxide are added. The resulting yellow solution is cooled to −55°C and a solution of 1.44 g (0.0055 mol) of 3-bromomethyl-2-chlorobenzo[b]thiophene in 10 ml of acetone is added. The mixture is stirred for 1 hour and 30 minutes allowing the temperature to reach room temperature. The solvent is evaporated in vacuo, the residue is dissolved in chloroform, washed neutral with water, dried sodium sulphate and evaporated. The crude product thus obtained is purified by silicagel column chromatography. 2.3 g of 1-(2,4-dichlorophenyl)-2-(1H, imidazol-1-yl)ethanone(Z)-O-(2-chloro-3-benzo[b]thienyl)methyl oxime (oily product) are obtained. Yield 84%.

Example 8

Bis[1-(2,4-dichlorophenyl)-2-(1H-imidazol-1-yl)ethanone] (Z)-O-[(2-chloro-3-benzo[b]thienyl)methyl]oxime naphthalen-1,5-disulphonate

(I, [A] = —C—, $R_1$ = 2—Cl, $R_2$ = H, 3 position, $\frac{1}{2}$ $C_{10}H_8O_6S_2$)
‖
N
\

As described in Example 2 and from the compound obtained in Example 7, Bis[1-(2,4-dichlorophenyl)-2-(1H-imidazol-1-yl)ethanone] (Z)-O-[(2-chloro-3-benzo[b]thienyl)methyl]oxime naphthalen-1,5-disulphonate, m.p. 218—220°C (ethanol), is obtained.

Example 9

1-(2,4-Dichlorophenyl)-2-(1H-imidazol-1-yl)ethanone (Z)-O-[(2-chloro-3-benzo[b]thienyl)-methyl]oxime mononitrate

(I, [A] = —C—, $R_1$ = 2—Cl, $R_2$ = H, 3 position, $NO_3H$)
‖
N
\

As described in Example 5 and from the compound obtained in Example 7, 1-(2,4-dichlorophenyl)-2-(1H-imidazol-1-yl)ethanone (Z)-O-[(2-chloro-3-benzo[b]thienyl)methyl]oxime mononitrate, m.p. 140—141°C (n-butanol), is obtained.

Example 10

1-[2-[(4-Fluoro-3-benzo[b]thienyl)methoxy]-2-(2,4-dichlorophenyl)ethyl]-1H-imidazol

(I, [A] = —CH—, $R_1$ = 4—F, $R_2$ = H, 3 position)
|

A 100 ml flask subjected to an inert and dry atmosphere is charged with 0.044 g of suspension of sodium hydride in (55%) paraffin oil (0.0018 mol) which is then washed three times with 5 ml of anhydrous hexamethylphosphorotriamide. Thereafter, 5 ml of hexamethylphosphorotriamide are added, cooled to 0°C and then a solution of 0.222 (0.086 mmol) of α-(2,4-dichlorophenyl)-1H-imidazol-1-ethanol in 8 ml of hexamethylphosphorotriamide is added. The mixture is stirred at room temperature until the volution of hydrogen stops, and allowed to stand at 50°C for 1 hour. Then the mixture is cooled again to 0°C and a solution of 0.235 g (0.96 mol) of 3-bromomethyl-4-fluorobenzo[b]thiophene in 5 ml of hexamethylphosphorotriamide is gently added, and allowed to stand at room temperature for 5 hours under stirring. After adding water and diethyl ether, the aqueous phase is extracted several times with ether, the organic phases are washed neutral with water, dried over sodium sulphate and evaporated in vacuo. The residue is purified by silicagel column chromatography yielding 0.236 g (65%) of 1-[2-[(4-fluoro-3-benzo[b]thienyl)methoxy]-2-(2,4-dichlorophenyl)ethyl]-1H-imidazol, m.p. 110—112°C.

$^1$H-NMR ($CDCl_3$): δ 4.0—4.2 (m, 2H), AB system centred at 4.72 (J ca 13Hz, 2H), 5.11 (dd, J ca 7 and 4Hz, 1H), 6.89 (s, 1H), 6.95 (s, 1H), 7.0—7.8 (m, 8H).

Example 11

1-[2-[(4-Fluoro-3-benzo[b]thienyl)methoxy]-2-(2,4-dichlorophenyl)ethyl]-1H-imidazol mononitrate

(I, [A] = —CH—, $R_1$ = 4—F, $R_2$ = H, 3 position, $NO_3H$)
|

To a solution of 0.436 g (1.035 mmol) of the compound as obtained in Example 10 in 5 ml of acetone 0.60 ml of 3N nitric acid are added. The mixture is concentrated to a volume of 2 ml and left to crystallize at 0°C 0.239 (48%) of 1-[2-[(4-fluoro-3-benzo[b]thienyl)methoxy]-2-(2,4-dichlorophenyl)ethyl]-1H-imidazol mononitrate of m.p. 150—151°C are obtained.

$^1$H-NMR ($CDCl_3$): δ 4.1—4.4 (m, 2H), 4.45 (d, J ca 12Hz, 1H), 4.80 (d, J ca 12Hz, 1H), 4.95—5.15 (m, 1H), 6.90 (s, wide, 1H), 7.10 (s, wide, 1H), 7.10—7.64 (m, 7H), 8.76 (s, 1H).

Example 12

1-[2-[(7-Chloro-3-benzo[b]thienyl)methoxy]-2-(2,4-dichlorophenyl)ethyl]-1H-imidazol

(I, [A] = —CH—, $R_1$ 7—Cl, = $R_2$ = H, 3 position)
|

A 100 ml flask subject to an inert and dry atmosphere is charged with a suspension of 0.142 g (0.00325 mol) of sodium hydride in 55% paraffin oil which is then washed three times with 5 ml of anhydrous hexamethylphosphorotriamide. Thereafter, 5 ml of hexamethylphosphorotriamide are added, cooled to 0°C and the mixture is then added to a solution of 0.771 g (3.0 mmol) of α-(2,4-dichlorophenyl)-1H-imidazol-1-ethanol in 15 ml of hexamethylphosphorotriamide. It is stirred at room temperature until the evolution of hydrogen stops and left to stand at 50°C for 1 hour. Thereafter, the mixture is cooled again to 0°C and a solution of 0.837 g (3.2 mmol) of 3-bromomethyl-4-chlorobenzo[b]thiophene in 15 ml of hexamethylphosphorotriamide is added, and left to stand at room temperature for 5 hours under stirring. After adding water and diethyl ether, the aqueous phase is extracted several times with ether. The organic phases are washed neutral with water, dried over sodium sulphate and evaporated in vacuo. The residue is purified by silicagel column chromatography yielding 0.805 g (62%) of 1-[2-[(7-chloro-3-benzo[b]thienyl)methoxy]-2-(2,4-dichlorophenyl)ethyl]-1H-imidazol, m.p. 146—147°C.

$^1$H-NMR ($CDCl_3$): δ 3.95—4.15 (m, 2H), AB system centred at 4.5 (J ca 11Hz, 2H), 4.95 (dd, J ca 6 and 4Hz, 1H), 6.7 (s, 1H), 6.8 (s, 1H), 7.1—7.5 (m, 8H).

### Example 13

1-[2-[(2-Benzo[b]thienyl)methoxy]-2-(2,4-dichlorophenyl)ethyl]-1H-imidazol mononitrate

(I, [A] = —CH—, $R_1$ = $R_2$ = H, 2 position, $NO_3H$)
|

To a solution of 1.40 g (3.55 mmol) of the compound as obtained in Example 3 in 5 ml of acetone, 1.5 ml of 3N nitric acid are added at 0°C. After crystallization at 0°C, 1.31 g (81%) of 1-[2-[(2-benzo[b]thienyl)methoxy]-2-(2,4-dichlorophenyl)ethyl]-1H-imidazol, m.p. 149—151°C, are obtained.

$^1$H-NMR ($d_6$-DMSO): δ 4.2—4.6 (m, 4H), 5.0 (t, J ca 6Hz, 1H), 7.1—8.0 (m, 10H), 9.1 (s, wide, 1H).

### Example 14

1-[2-[(5-Chloro-3-benzo[b]thienyl)methoxy]-2-(2,4-dichlorophenyl)ethyl]-1H-imidazol

(I, [A] = —CH—, $R_1$ = 5—Cl, $R_2$ = H, 2 position)
|

A 100 ml flask subject to an inert and dry atmosphere is charged with a suspension of 0.070 g (1.60 mmol) of sodium hydride in 55% paraffin oil which is then washed three times with 5 ml of anhydrous hexamethylphosphorotriamide. Thereafter, 5 ml of hexamethylphosphorotriamide are added, cooled to 0°C and then a solution of 0.372 (1.447 mmol) of α-(2,4-dichlorophenyl)-1H-imidazol-1-ethanol in 5 ml of hexamethylphosphorotriamide is added. The mixture is stirred at room temperature until the evolution of hydrogen stops, and allowed to stand at 50°C for 1 hour. Thereafter, the mixture is cooled again to 0°C and a solution of 0.420 g (1.60 mmol) of 2-bromomethyl-5-chlorobenzo[b]thiophene in 5 ml of hexamethylenephosphorotriamide is carefully added, and the mixture is allowed to stand at room temperature for 5 hours. After adding water and diethyl ether, the aqueous phase is extracted several times with ether; the organic phases are washed neutral with water, dried over sodium sulphate and evaporated in vacuo. The residue is purified by silicagel column chromatography yielding 0.234 g (37%) of 1-[2-[(5-chloro-2-benzo[b]thienyl)methoxy]-2-(2,4-dichlorophenyl)ethyl]-1H-imidazol (oily product).

$^1$H-NMR ($CDCl_3$): δ 4.04—4.25 (m, 2H), AB system centred at 4.6 (J ca 12 Hz, 2H), 5.1 (dd, J ca 6 and 4Hz, 1H), 6.9—7.9 (m, 10H).

### Example 15

1-(2,4-Dichlorophenyl)-2-(1H-imidazol-1-yl)ethanone (Z)-O-[(5-chloro-2-benzo[b]thienyl)methyl]oxime

(I, [A] = —C—, $R_1$ = 5Cl, $R_2$ = H, 2 position)
‖
N
\

In a 25-ml flask 1.89 g (0.0070 mol) of 1-(2,4-dichlorophenyl)-2-(1H-imidazol-1-yl)-ethanone(Z)oxime are suspended in 6 ml of acetone, then 0.507 g (0.0077 mol) of 85% triturated potassium hydroxide are added under stirring. The resulting yellow solution is cooled at −55°C and a solution of 1.44 g (0.0055 mol) of 2-bromomethyl-5-chlorobenzo[b]thiophene in 15 ml of acetone is added. The mixture is stirred for 1 hour and 30 minutes allowing the temperature to reach room temperature. The solvent is evaporated in vacuo, the residue is dissolved in chloroform, washed netural with water, dried sodium sulphate and evaporated. The crude product thus obtained is purified by silicagel column chromatography. 2.21 g (70%) of 1-(2,4-dichlorophenyl)-2-(1H-imidazol-1-yl)ethanone (Z)-O-[(2-chloro-3-benzo[b]thienyl)methyl] oxime (oily product) are obtained.

$^1$H-NMR ($CDCl_3$): δ 5.35 (s, 2H), 5.50 (s, 2H), 6.8 (s, wide, 1H), 6.95 (s, wide, 1H), 7.1—8.0 (m, 8H).

### Example 16

Bis[1-(2,4-dichlorophenyl)-2-(1H-imidazol-1-yl)ethanone] (Z)-O-[(5-chloro-2-benzo[b]thienyl)methyl]oxime naphthalen-1,5-disulphonate

(I, [A] = —C—, $R_1$ = 5—Cl, $R_2$ = H, 3 position, $\frac{1}{2}$ $C_{10}H_8O_6S_2$)
‖
N
\

A solution of 1.11 g (0.00246 mol) of the compound as obtained in Example 15 in 30 ml of ethanol is added to a solution of 0.475 g (75.8%) of 1,5-naphthalendisulphonic acid (0.00125 mol) in 50 ml of ethanol. 0.731 g (50%) of Bis[1-(2,4-dichlorophenyl)-2-(1H-imidazol-1-yl)ethanone] (Z)-O-[(5-chloro-2-benzo[b]thienyl)methyl]oxime naphthalen-1,5-disulphate, m.p. 174—175°C, are obtained.

Example 17

1-[2-[(5-fluoro-2-benzo[b]thienyl)methoxy]-2-(2,4-dichlorophenyl)ethyl]-1H-imidazol
(I, [A] = —CH—, $R_1$ = 5—F, $R_2$ = H, 2 position)

A 250 ml flask subject to an inert and dry atmosphere, is charged with 0.132 g of suspension of sodium hydride in 55% paraffin oil (0.003 mol) which is then washed three times with 15 ml of anhydrous hexamethylphosphorotriamide. Thereafter, 15 ml of hexamethylphosphorotriamide are added, cooled to 0°C and then a solution of 0.720 (2.8 mmol) of α-(2,4-dichlorophenyl)-1H-imidazol-1-ethanol in 20 ml of hexamethylphosphorotriamide is added. The mixture is stirred at room temperature until the evolution of hydrogen stops, and allowed to stand at 50°C for 1 hour. Then the mixture is cooled again to 0°C and a solution of 0.700 g (2.85 mmol) of 2-bromomethyl-5-fluorobenzo[b]thiophene in 15 ml of hexamethylenephosphorotriamide is gently added, and allowed to stand at room temperature for 5 hours under stirring. After adding water and diethyl ether, the aqueous phase is extracted several times with ether, the organic phases are washed neutral with water, dried over sodium sulphate and evaporated in vacuo. The residue is purified by silicagel column chromatography yielding 0.334 g (29%) of 1-[2-[(5-fluoro-2-benzo[b]thienyl)methoxy]-2-(2,4-dichlorophenyl)ethyl]-1H-imidazol (oily product).

$^1$H-NMR ($CDCl_3$): δ 4.04—4.2 (m, 2H), AB systemn centred at 4.6 (J ca 12Hz, 2H), 5.1 (dd, J ca 7 and 4Hz, 1H), 6.9—7.9 (m, 10H).

Example 18

1-[2-[(5-Fluoro-2-benzo[b]thienyl)methoxy]-2-(2,4-dichlorophenyl)ethyl]-1H-imidazol mononitrate
(I, [A] = —CH—, $R_1$ = 5—F, $R_2$ = H, 2 position, $NO_3H$)

To a solution of 0.090 g (0.213 mmol) of the compound as obtained in Example 17 in 3 ml of acetone, 0.2 ml of 3N nitric acid are added. The mixture is concentrated to a volume of 2 ml and left to crystallize at 0°C. 0.050 (48%) of 1-[2-[(5-fluoro-2-benzo[b]thienyl)methoxy]-2-(2,4-dichlorophenyl)ethyl]-1H-imidazol mononitrate, m.p. 164—166°C, are obtained.

$^1$H-NMR ($D_6$-DMSO): δ 4.45—5.0 (m, 4H), 5.24 (t, J ca 7Hz, 1H), 7.1—8.0 (m, 9H), 9.0; (s, 1H).

Example 19

1-[2-[(7-Chloro-3-benzo[b]thienyl)methoxy]-2-(2,4-dichlorophenyl)ethyl]-1H-imidazol mononitrate
(I, [A] = —CH—, $R_1$ = 7—Cl, $R_2$ = H, 3 position, $NO_3H$)

To a solution of 1.30 g (2.97 mmol) of the compound as obtained in Example 12 in 30 ml of 3N nitric acid are added. The mixture is concentrated to a volume of 10 ml and left to crystallize at 0°C. 1.24 g of 1-[2-[(7-chloro-3-benzo[b]thienyl)methoxy]-2-(2,4-dichlorophenyl)ethyl]-1H-imidazol mononitrate, m.p. 156—157°C, are obtained. Yield 84%.

$^1$N-NMR ($D_6$-DMSO): δ 4.5 (d, J ca (5Hz, 2H), AB system centred at (4.7 (J ca 12.8 Hz, 2H), 5.2 (t, J ca 5Hz, 1H), 7.2—7.75 (m, 9H), 9.0 (s, 1H).

Example 20

1-[2-[(5-Chloro-2-benzo[b]thienyl)methoxy]-2-(2,4-dichlorophenyl)ethyl]-1H-imidazol mononitrate
(I, [A] = —CH—, $R_1$ = 5—Cl, $R_2$ = H, 2 position, $NO_3H$)

To a solution of 0.810 g (1.85 mmol) of the compound as obtained in Example 14 in 5 ml of acetone, 1.0 ml of 3N nitric acid are added at 0°C. The mixture is concentrated to a volume of 3 ml and left to crystallize at 0°C. 0.754 g of 1-[2-[(5-chloro-3-benzo[b]thienyl)methoxy]-2-(2,4-dichlorophenyl)ethyl]-1H-imidazol mononitrate, m.p. 153—155°C, are obtained. Yield 81%.

$^1$H-NMR ($D_6$-acetone): δ 4.6—5.0 (m, 4H), 5.4 (dd, J ca 4.4 and 7.1 Hz, 1H), 7.2—8.0 (m, 9H), 8.95 (s, 1H).

Examples 21—43

The compounds listed below (Table 2) are obtained as described in the aforesaid examples using respective synthesis intermediates.

TABLE 2

| Example No. | Compund (I) | | | | | |
|---|---|---|---|---|---|---|
| | A | $R_1$ | $R_2$ | Position | Base or Salt | Melting point °C |
| 21 | -C-<br>‖<br>N\ | H | H | 2 | Base | oil |
| 22 | -C-<br>‖<br>N\ | H | H | 2 | Naphthalen-1,5-disulphonate (Base-Acid 1:$\frac{1}{2}$) | 184-186 |
| 23 | -C-<br>‖<br>N\ | 5-Cl | H | 3 | Base | oil |
| 24 | -C-<br>‖<br>N\ | 5-Cl | H | 3 | Naphthalen-1,5-disulphonate (Base-Acid 1:$\frac{1}{2}$) | 198-200 |
| 25 | -C-<br>‖<br>N\ | 4-Cl | H | 3 | Base | 155-156 |
| 26 | -C-<br>‖<br>N\ | 6-Cl | H | 3 | Base | oil |
| 27 | -C-<br>‖<br>N\ | 6-Cl | H | 3 | Naphthalen-1,5-disulphonate (Base-Acid 1:$\frac{1}{2}$) | 123-125 |
| 28 | -C-<br>‖<br>N\ | 5-F | H | 3 | Base | oil* |
| 29 | -CH-<br>\| | 5-Cl | H | 3 | Base | 163-165 |
| 30 | -CH-<br>\| | 2-Cl | H | 3 | Base | 155-156 |
| 31 | -CH-<br>\| | 6-Cl | H | 3 | Base | 160-161 |
| 32 | -CH-<br>\| | 5-F | H | 3 | Base | 180-181 |
| 33 | -CH-<br>\| | 6-F | H | 3 | Base | 150-152 |
| 34 | -CH-<br>\| | 4-Cl | 6-Cl | 3 | Base | 136-138 |

TABLE 2 (cont.)

| Example No. | A | $R_1$ | $R_2$ | Compound (I) Position | Base or Salt | Melting point °C |
|---|---|---|---|---|---|---|
| 35 | -CH- | 4-Br | H | 3 | Base | oil |
| 36 | -CH- | 4-Br | H | 3 | Naphthalen-1,5-disulphonate (Base-Acid 1:$\frac{1}{2}$) | 212-215 |
| 37 | -CH- | 6-Br | H | 3 | Base | 139-141 |
| 38 | -C- ‖ N | 7-Cl | H | 3 | Base | oil |
| 39 | -C- ‖ N | 7-Cl | H | 3 | Naphthalen-1,5-disulphonate (Base-Acid 1:$\frac{1}{2}$) | 212-214 |
| 40 | -CH- | 7-F | H | 3 | Base | 135-137 |
| 41 | -C- ‖ N | 7-F | H | 3 | Base | oil** |
| 42 | -CH- | 7-Cl | H | 2 | Base | oil*** |
| 43 | -CH- | 7-F | H | 2 | Base | oil**** |

* $^1$H-NMR ($CDCl_3$): 5.10 (s,2H), 5.40 (s,2H), 6.57 (s, wide,1H), 6.80 (s,wide,1H), 6.9-7.95 (m,8H).

** $^1$H-NMR ($CDCl_3$): 5.10 (s,2H), 5.45 (s,2H), 6.55 (s, wide,1H), 6.80 (s,wide,1H), 6.9-7.8 (m,8H).

*** $^1$H-NMR ($CDCl_3$): 3.95-4.05 (m,2H), AB system centered at 4.45 (J ca 12Hz,2H), 4.9 (dd, J ca 7 and 4Hz, 1H), 6.9-7.85 (m, 10H).

**** $^1$H-NMR ($CDCl_3$): 3.95-4.05 (m,2H), AB system centered at 4.45 (J ca 12Hz,2H), 4.95 (dd, J ca 7 and 3Hz,1H), 6.8-7.5 (m, 10H).

**Claims**

1. 1H-imidazole derivatives having the general formula (I):

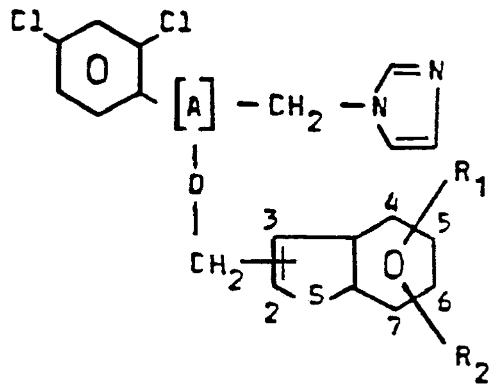

(I)

wherein [A] is an imino-

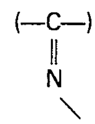

or methine-

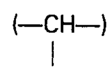

group,

$R_1$ and $R_2$, which may be the same or different, are hydrogen or halogen, the halogen being attached to the benzo[b]-thiophene group in the 2, 4, 5, 6 or 7 position and the methylene ($CH_2$—) group being bonded to the benzo[b]thiophene group in 2 or 3 position, and the nontoxic addition salts thereof.

2. A process for preparing the compounds according to Claim 1, characterized in that a compound of the general formula (II):

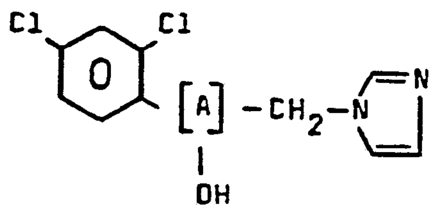

(II)

wherein [A] has the same meaning as defined in claim 1, is reacted with a compound of the general formula (III):

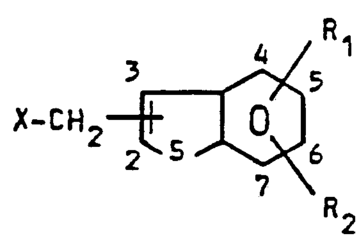

(III)

wherein X is chlorine or bromine, $R_1$ and $R_2$ are as defined in claim 1 and the methylene (—$CH_2$—) group is also bonded to the benzo[b]thiophene group in the same positions as defined in claim 1, in a medium constituted by an aprotic solvent selected from an alkanone with up to 6 carbon atoms, or a polyalkylated amide, and in the presence of a mineral base, and, if desired, the so obtained free base is converted to an addition salt thereof by treating it with respective acid in a medium constituted by an organic solvent selected from an alkanol or alkanone having 1 to 4 carbon atoms or mixture thereof with water.

3. The process of Claim 2, characterized in that acetone of hexamethylphosphorotriamide is used as aprotic solvent.

4. The process of Claim 2, characterized in that potassium hydroxide or sodium hydride is used as mineral base.

5. The process of Claim 2, characterized in that ethanol, n-butanol or acetone is used as organic solvent.

6. The process of any one of the preceding claims, characterized in that the reaction is carried out at a temperature of −55 to 100°C.

7. Pharmaceutical composition comprising at least one of the compounds of Claim 1, optionally together with pharmaceutical carriers and/or adjuvants.

8. The use of the compounds according to claim 1 for the preparation of pharmaceutical compositions for treating infections in humans and animals or combatting crop diseases.

**Patentansprüche**

1. 1H-Imidazol Derivate der allgemeinen Formel (I):

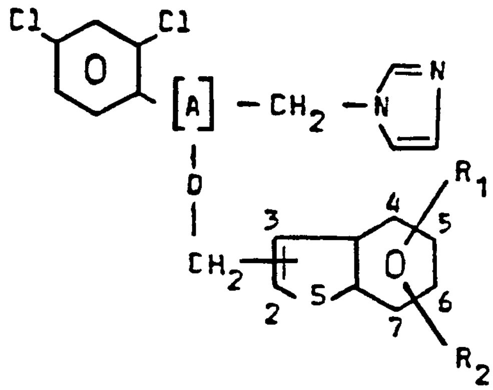

(I)

worin [A] für eine Imino-

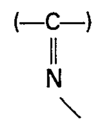

oder Methin-

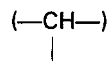 gruppe steht,

$R_1$ und $R_2$, die gleich oder verschieden sein können, für ein Wasserstoff- oder ein Halogenatom stehen, wobei das halogenatom an die Benzo[b]-Thiophengruppe in der 2-, 4-, 5-, 6- oder 7-Stellung und die Methylen-($-CH_2-$)-Gruppe an die Benzo[b]-Thiophengruppe in 2- oder 3-Stellung gebunden ist und die nicht-toxischen Additionssalze davon.

2. Verfahren zur Herstellung der Verbindungen nach Anspruch 1, dadurch gekennzeichnet, daß eine Verbindung der allgemeinen Formel (II):

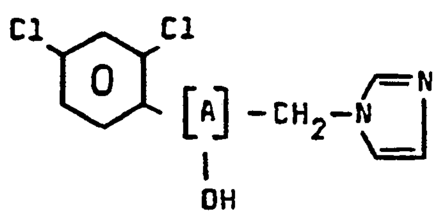

(II)

worin [A] die gleiche Bedeutung wie in Anspruch 1 besitzt, mit einer Verbindung der allgemeinen Formel (III)

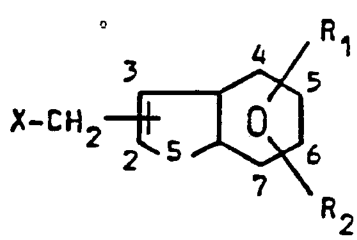

(III)

worin X für Chlor oder Brom steht, $R_1$ und $R_2$ wie in Anspruch 1 definiert sind, und die Methylen ($-CH_2-$) gruppe in den gleichen Stellungen wie in Anspruch 1 definiert an die Benzo[b]thiophengruppe gebunden ist, in einem Medium, bestehend aus einem aprotischen Lösungsmittel ausgewählt unter einem Alkanon mit bis zu 6 Kohlenstoffatomen, oder einem polyalkylierten Amid, und in Anwesenheit einer mineralischen Base umgesetzt wird, und falls gewünscht, die so erhaltene freie Base, durch Behandlung mit der entsprechenden Säure in einem Medium, bestehend aus einem organischen Lösungsmittel ausgewählt unter einem Alkanol oder Alkanon mit 1 bis 4 Kohlenstoffatomen oder Mischungen davon mit Wasser, in ein Additionssalz davon umgewandelt wird.

3. Verfahren nach Anspruch 2, dadurch gekennzeichnet, daß Aceton oder Hexamethylphosphortriamid als aprotisches Lösungsmittel verwendet wird.

4. Verfahren nach Anspruch 2, dadurch gekennzeichnet, daß Kaliumhydroxid oder Natriumhydrid als mineralische Base verwendet wird.

5. Verfahren nach Anspruch 2, dadurch gekennzeichnet, daß Äthanol, n-Butanol oder Aceton als organisches Lösungsmittel verwendet wird.

6. Verfahren nach einem der vorhergehenden Ansprüche, dadurch gekennzeichnet, daß die Reaktion bei einer Temperatur von −55 bis 100 C durchgeführt wird.

7. Pharmazeutische Zusammensetzung enthaltend zeumindest eine Verbindung nach Anspruch 1, gegebenenfalls zusammen mit pharmazeutischen Trägern und/oder Adjuvantien.

8. Verwendung der Verbindungen nach Anspruch 1 zur Herstellung von pharmazeutischen Zusammensetzungen zur Behandlung von Infektionen bei Menschen und Tieren oder zur Bekämpfung von Getreidekrankheiten.

## Revendications

1. Dérivés du 1H-imidazole répondant à la formule générale (I):

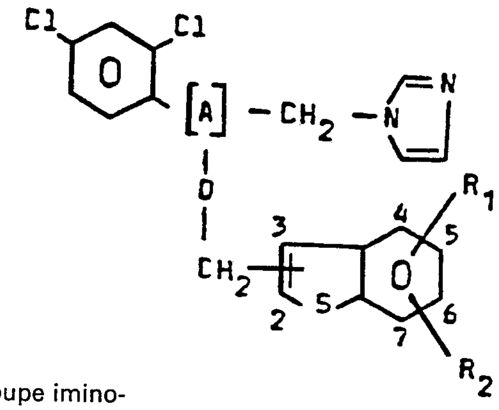

(I)

dans laquelle [A] est un groupe imino-

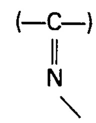

ou méthine-

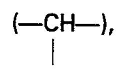

$R_1$ et $R_2$ qui peuvent être identiques ou différents sont des atomes d'hydrogène ou d'halogène, l'halogène étant fixé sur le groupe benzo[b]-thiophène en position 2, 4, 5, 6 ou 7 et le groupe méthylène ($-CH_2-$) étant lié au groupe benzo[b]-thiophène en position 2 ou 3, et leurs sels d'addition non toxiques.

2. Procédé de prépartion des composés selon la revendication 1, caractérisé en ce qu'on fait réagir un composé répondant à la formule générale (II):

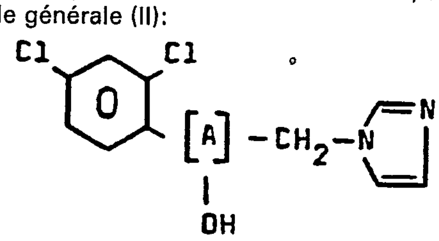

(II)

dans laquelle [A] a la même signification que dans la revendication 1, avec une composé répondant à la formule générale (III):

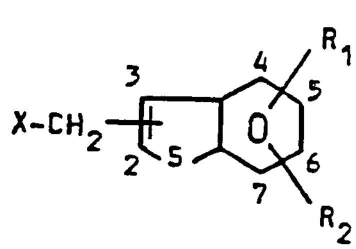

(III)

dans laquelle X est un atome de chlore ou de brome, $R_1$ et $R_2$ sont tels que définis dans la revendication 1, et le groupe méthylène ($—CH_2—$) et également lié au groupe benzo[b]thiophène dans les mêmes positions que dans la revendication 1, dans un milieu constitué d'un solvant aprotique choisi parmi une alcanone ayant jusqu'à 6 atomes de carbone, ou un amide polyalkylé, et en présence d'une base minérale et, si on le désire, on transforme la base libre ainsi obtenue en un sel d'addition de celle-ci en la traitant pr l'acide respectif dans un milieu constitué par un solvant organique choisi par un alcanol ou une alcanone en $C_1$ à $C_4$ ou des mélanges de ceux-ci avec l'eau.

3. Procédé selon la revendication 2, caractérisé en ce qu'on utilise comme solvant aprotique l'acétone ou l'hexaméthylphosphorotriamide.

4. Procédé selon la revendication 2, caractérisé en ce qu'on utilise comme base minérale l'hydroxyde de potassium ou l'hydrure de sodium.

5. Procédé selon la revendication 2, caractérisé en ce qu'on utilise comme solvant organique l'éthanol, le n-butanol ou l'acétone.

6. Procédé selon l'une quelconque des revendications précédentes, caractérisé en ce que la réaction est effectuée à une température de −55 à 100°C.

7. Composition pharmaceutique comprenant au moins un des composés de la revendication 1, avec si on le désire des supports et/ou adjuvants pharmaceutiques.

8. Utilisation des composés selon la revendication 1, pour la préparation de compositions pharmaceutiques pour traiter des infections chez l'homme et l'animal ou pour combattre des maladies de récoltes.